# EUROPÄISCHE PATENTANMELDUNG

(11) **EP 1 481 988 A2**
(43) Veröffentlichungstag der Anmeldung: **01.12.2004**
(21) Anmeldenummer: 04019254.4
(22) Anmeldetag: 08.06.2001
(51) Int. Cl.: C07K 14/47

(54) **Verfahren zur Gewinnung und Anwendung von antibiotisch wirksamen Peptiden zur Behandlung von Infektionskrankheiten**

(30) Priorität: 08.06.2000 DE 10027992
(62) Teilanmeldung aus: 01960288.7
(71) Anmelder: IPF PharmaCeuticals GmbH, 30625 Hannover (DE)
(72) Erfinder: Forssmann, Wolf-Georg, 30625 Hannover (DE); Liepke, Cornelia, 30625 Hannover (DE); Arndt, Susann, 30625 Hannover (DE)
(74) Vertreter: von Kreisler Selting Werner

(57) **Zusammenfassung**

Die Erfindung betrifft antibiotisch wirksame Peptide, die zum Zwecke der medizinischen und gewerblichen Verwendung als Arzneimittel aus humanen und tierischen Gewebeextrakten gereinigt wurden. Die Peptide können mittels biotechnologischer und rekombinanter Verfahren und chemischer Synthese sowie aus korrespondierenden Vorläuferproteinen proteolytisch hergestellt werden. Die antibiotisch wirksamen Peptide können in geeigneter galenischer Zubereitung als Medikamente/Tiermedikamente und Lebensmittelzusätze verwendet werden.

## Beschreibung

Die Erfindung betrifft antibiotische Peptide, Verfahren zu ihrer Herstellung sowie ihre Verwendung.

Es ist bekannt, dass natürlich vorkommende Peptide antibiotisch wirksam sein können. So beschreibt die WO 97/35877 das Vorkommen und die Aufreinigung von antibiotischen Peptiden aus Kuhmilch.

Überraschenderweise wurde nun gefunden, dass antibiotisch aktive Peptide auch aus humanem Plazentaextrakt und aus bovinem Thymusextrakt gewonnen werden können.

Dazu wurden die genannten Gewebeextrakte chromatographisch aufgereinigt, und die erhaltenen Fraktionen unter Anwendung des Radial-Diffusions-Hemmtests auf antimikrobielle Aktivität überprüft. Überraschenderweise wurde festgestellt, dass in den Fraktionen Substanzen enthalten sind, die das Wachstum von Bakterien stark hemmen. Durch weitere chromatographische Trennverfahren konnten die biologisch aktiven Substanzen konzentriert, zur Homogenität gereinigt und ihre Struktur aufgeklärt werden.

Die gereinigten Peptide wirken überraschenderweise sowohl auf Gram-positive Bakterien wie *Bacillus subtilis, Micrococcus luteus* und *Staphylococcus carnosus* als auch auf Gram-negative Bakterien wie *Escherichia coli* und *Pseudomonas fluorescens* stark wachstumshemmend. Die wachstumsmodulierende Eigenschaft der gereinigten Peptide auf Mikroorganismen wurde bei der Entwicklung des hier vorgestellten Verfahrens erstmals nachgewiesen.

Die vorliegende Erfindung betrifft folglich neue antimikrobiell wirksame Peptide, ihre Herstellung, diese Peptide enthaltende Arzneimittel sowie diese Peptide enthaltende Zubereitungen und ihre Verwendung.

Die vorliegende Erfindung betrifft Peptide mit der Aminosäuresequenz

| | |
|---|---|
| 1. | R₁-WQKMVTAVASALSSRYH-R₂ |
| 2. | R₁-GKVKVGVNGFGRIGRLVTRAAFNSGKVDIVA-R₂ |
| 3. | R₁-VCVLAHHFGKEFTPPVQAAYQKWAGVANALAHKYH-R₂ |
| 4. | R₁-KAGAHLKGGAKRVIISAPS-R₂ |
| 5. | R₁-YIVYKIRSADKRSKALK-R₂ |
| 6. | R₁-KAASKKAPSKASPNPRSGTRRSP-R₂ |
| 7. | R₁-KAASKKAPSKASPNPRSGTRRSPRIATTAASAATAAASAAA TTPRAKGKAKH -R₂ |
| 8. | R₁-CAIHAKRVTIMPKDIQLARRIRGERA-R₂ |
| 9. | R₁-LSKGLIKLVSKHRAQVIYTRNTKGGDAPAAG-R₂ |
| 10. | R₁-QQKQQKGRGMGGAGRGVFGGRGRGGIPGTGRG QPEKKPG-R₂ |
| 11. | R₁-LKSLVSKGTLVQTKGTGASGSFKLNKKAATGEAK PKGKKAGAAKPKKAAGAAKKPKK-R₂ |
| 12. | R₁-SSGEEVVIPQKKGKKAATTPAKKMWSPTKKVAVAT PAKKAVVTPGKKAAAMPAKKTVTPAKAVVTPGKKGATPGKA WATSGKKGAATPGKGAKNGKNAKKED-R₂ |

worin R₁ eine Aminogruppe (NH₂), eine Aminosäure oder ein Peptid bis zu 50 Aminosäuren darstellt und R₂ COOH, CONH₂, eine Aminosäure oder ein Peptid mit bis zu 50 Aminosäuren darstellt, sowie die amidierten, acetylierten, sulfatierten, phosphorylierten, glycosylierten, oxydierten Derivate und weiterhin Fragmente mit antimikrobiellen Eigenschaften, die aus der Aminosäuresequenz abgeleitet werden. Bevorzugt weisen die Reste R₁ und R₂ unabhängig voneinander eine Länge von bis zum 40 Aminosäuren, bevorzugt bis zu 30 Aminosäuren, noch mehr bevorzugt bis zu 20 Aminosäuren und am meisten bevorzugt bis zu 10 Aminosäuren auf.

Neben den erwähnten Derivaten können die Peptide auch statt der natürlichen L-Aminosäuren D-Aminosäuren aufweisen, was den Abbau durch Proteasen verzögert.

Gegenstand der Erfindung sind auch Peptide, in denen einzelne Aminosäuren ausgetauscht sind, insbesondere durch sogenannte konservative Austausche innerhalb der bekannten Gruppen von sauren, basischen, neutralen, hydrophoben, aromatischen und hydrophilen Aminosäuren. Beispiele hierfür sind Serin gegen Alanin oder Alanin gegen Serin, Isoleucin gegen Leucin oder Leucin gegen Isoleucin usw.

Als Fragmente kommen insbesondere N- oder C-terminal verkürzte Peptide in Frage, aber auch Peptide, in denen einzelne Aminosäuren deletiert sind, vorzugsweise nicht mehr als 10 % der Aminosäuren. Diese Fragmente sind insoweit Gegenstand der Erfindung, als sie antimikrobielle Eigenschaften aufweisen. Antimikrobielle Eigenschaften des Fragmentes bedeutet, dass das Fragment mindestens 50 % der Aktivität des Stammpeptides hat, also maximal die doppelte Hemmkonzentration benötigt. Dabei werden als Testmikroorganismen entweder Staphylococcus aureus oder Escherichia coli verwendet, je nachdem, bei welchem Mikroorganismus das Peptid bessere Wirksamkeit hat. Die Bakterien sind wie folgt charakterisiert: Staphylococcous aureus: Gram-positive Kokken, ATCC 25923 Escherichia coli: Gram-negative Stäbchen, ATCC 25922 Die minimale Inhibitionskonzentration wurde in Anlehnung an die von dem NCCLS (National Committee for Clinical Laboratory Standards) herausgegebenen Empfehlungen (M7-A3) analog Beispiel 4 bestimmt.

Die erfindungsgemäßen Peptide können durch Isolierung aus menschlichen und tierischen Gewebeextrakten, durch Proteolyse aus korrespondierenden Vorläuferproteinen, durch chemische und biotechnologische Synthese in reiner und biologisch aktiver Form hergestellt werden.

Bei den erfindungsgemäßen Peptiden handelt es sich um Fragmente von größeren Vorläuferproteinen. Dies ist genauer in der nachfolgenden Tabelle dargestellt.

| Vorläuferprotein | Peptid Seq ID No. |
|---|---|
| aus humaner Placenta | |
| Glyceraldehyde-3-phosphate Dehydrogenase (GAPDH) | 2 |
| Hämoglobin b | 3 |
| fetales Hämoglobin g | 1 |
| Histon H3 | 8 |
| 40 S Ribosomal Protein S25 | 9 |
| aus Kalbsthymus | |
| mitochondriales Proteolipid | 5 |
| GAPDH | 4 |
| glycine-rich protein | 10 |
| Nuclear mitotic apparatus protein | 6, 7 |
| Histon H1 | 11 |
| Nucleolin | 12 |

Die erfindungsgemäßen Peptide eignen sich zur Behandlung von durch mikrobielle Fehlbesiedlungen bedingten Erkrankungen wie Infektionen, Entzündungen, mikrobiell induzierten Tumoren, mikrobiell bedingten degenerativen Erkrankungen, Durchfallerkrankungen, Koliken, Abweichungen der Mund-, Darm- und Vaginalflora, Karies. Die mikrobielle Fehlbesiedlung kann beispielsweise durch Bakterien, Pilze, Hefen, Protisten, Viren, Mycoplasmen, Filarien und/oder Plasmodien bedingt sein. Sie können sowohl bei akuten als auch bei chronischen Erkrankungen eingesetzt werden.

Die Arzneimittelzubereitung enthält die erfindungsgemäßen Peptide bzw. ein physiologisch verträgliches Salz der Peptide. Die Form und Zusammensetzung des Arzneimittels, welches die Peptide enthält, richtet sich nach der Art der Verabreichung. Bevorzugt werden galenische Zubereitungen eingesetzt und Applikationsformen gewählt, bei denen die Peptide undegradiert an den Wirkort gelangen. Arzneimittelzubereitungen können pharmazeutisch übliche Hilfsstoffe, die z. B. einen Beitrag zur Löslichkeit, Stabilität oder Sterilität des Arzneimittels leisten oder den Wirkungsgrad der Aufnahme in den Körper erhöhen, enthalten. Die Peptide können parenteral, intranasal, oral und mittels Inhalation verabreicht werden. Vorzugsweise werden die Peptide zu einem Injektionspräparat, entweder als Lösung oder als Lyophilisat zur Auflösung unmittelbar vor Gebrauch, konfektioniert.

Die zu verabreichende Dosis der Peptide hängt von der Indikation und der Anwendung bestimmter Derivate ab. Vorzugsweise werden die erfindungsgemäßen Peptide in Mengen von 0,1 bis 100 mg/kg Körpergewicht eingesetzt.

Die erfindungsgemäßen Peptide können auch als Zusatzstoffe für Lebensmittel verwendet werden. Sie können bei der Herstellung von Lebensmitteln als Hilfsstoffe, insbesondere bei Lebensmitteln, die durch Gärung und andere bakterielle Prozesse hergestellt werden, verwendet werden. Bei den erfindungsgemäßen Präparaten handelt es sich um natürliche Konservierungsmittel.

Die folgenden Beispiele sollen die Erfindung weiter erläutern:

### Beispiel 1

### Herstellung des Plazentaextrakts

Humane Plazenta wurde mit Extraktionspuffer (1,0 M Essigsäure, 20 mM Ascorbinsäure, 1 mM EDTA, pH 2,45) und Eis homogenisiert und zur Extraktion über Nacht bei 4 °C gerührt. Das extrahierte Homogenisat wurde zentrifugiert und der Überstand filtriert. Das erhaltene Filtrat wurde ultrafiltriert (50 kDa PS-Membran) und das Permeat nachfolgend auf einen Kationenaustauscher gegeben und stufenweise pH-Wert abhängig eluiert. In den Eluaten enthaltene Peptide/Proteine wurden über RP-Chromatographie weiter aufgetrennt.

### HPLC Reinigung von antimikrobiellen Peptiden aus Plazentaextrakt

Für die Reinigung von antimikrobiellen Peptiden aus Plazentaextrakt können verschiedene HPLC Trennverfahren kombiniert werden, um eine möglichst reine Darstellung über eine optimale Trennung zu erzielen und inaktive, unerwünschte Bestandteile abzutrennen. Die jeweiligen Proben, die nach jedem Trennungsschritt entstehen, werden auf ihre antimikrobielle Aktivität gegen *Escherichia coli* mit Hilfe des Radial-Diffusions-Hemmtests untersucht (siehe Beispiel 3). Notwendig für die Reinigung ist die Kombination von mindestens zwei Reverse Phase Chromatographieschritten und der Einsatz einer Kationenaustausch-HPLC-Trennung. In dem Biotest sollte die jeweilige Probe salzarm eingesetzt werden, um ein möglichst optimales Screeningergebnis zu erhalten.

Der erste Trennschritt wurde mit Hilfe einer Fineline RPC Polymer Säule (20 x 15,5 cm, 15 µm, Pharmacia Biotech, Freiburg, Deutschland) durchgeführt.

Puffer A: 10 mM HCl; Puffer B: 10 mM HCl / 80 % Acetonitril / 20 % bidestilliertes Wasser; Gradient: 0 -50 % B in 60 Minuten; Fluss: 400 ml/min; Detektion: 280 nm
Das Chromatogramm ist in Figur la dargestellt. Die Balken geben die antimikrobielle Aktivität der erhaltenen Fraktionen an.

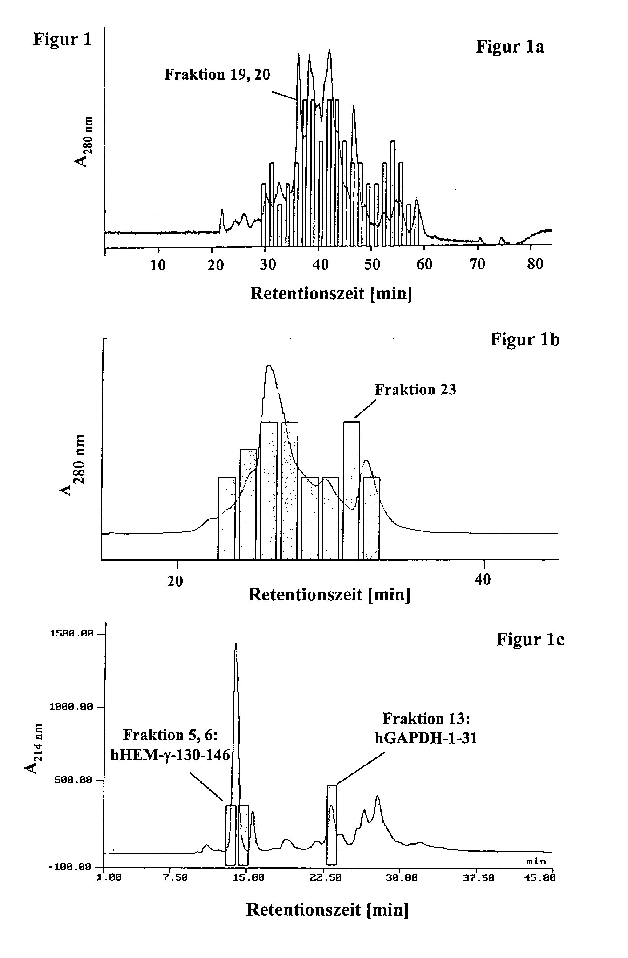

Rechromatographie von Fraktion 19 und 20 mit einer YMC Gel C8 - C18 Säule (47 x 300 mm, 15 µm, 300 Å, Waters, Milford, MA).
Puffer A: 10 mM HCl; Puffer B: 10 mM HCl / 80 % Acetonitril / 20 % bidestilliertes Wasser; Gradient: 20 - 70 % B in 37,5 Minuten; Fluss: 40 ml/min; Detektion: 280 nm
Das Chromatogramm ist in Figur 1b dargestellt. Die Balken geben die antimikrobielle Aktivität der erhaltenen Fraktionen an.

Rechromatographie von Fraktion 23 des vorangegangenen Trennschrittes mit einer Kationenaustausch HPLC.
Säule: Parcosil Pepkat, 4 x 50 mm, 5 µm, 300 Å, Biotek, Heidelberg, Deutschland
Puffer A: 10 mM Natriumphosphatpuffer pH 7,2; Puffer B: Puffer A mit 1 M NaCl
Gradient: 0 - 60 % B in 60 Minuten; Fluss: 0,75 ml/min; Detektion: 214 nm.

Die erhaltenen Fraktionen wurden einzeln mit einem Reverse-Phase Chromatographieschritt entsalzt, bevor sie dem Test auf antimikrobielle Aktivität zugeführt wurden. Das Chromatogramm ist in Figur 1c dargestellt. Die Balken geben die antimikrobielle Aktivität der erhaltenen Fraktionen an.

Durch Massenspektrometrie und Aminosäuresequenzierung wurden die folgenden Peptide identifiziert:
Fraktion 5 + 6 enthielten das reine, antimikrobiell wirksame Peptid hHEM-g-130-146 mit der Aminosäuresequenz: WQKMVTAVASALSSRYH Seq ID No. 1
Fraktion 13 enthielt das reine, antimikrobiell wirksame Peptid hGAPDH-1-31 mit der Aminosäuresequenz: GKVKVGVNGFGRIGRLVTRAAFNSGKVDIVA Seq ID No. 2

### Beispiel 2

### Herstellung des Thymusextrakts

Kalbsthymus wurde mit Extraktionspuffer (1,0 M Essigsäure, 10 mM Ascorbinsäure, 0,5 mM EDTA) und Eis homogenisiert und zur Extraktion über Nacht bei 4 °C gerührt. Das extrahierte Homogenisat wurde zentrifugiert und der Überstand filtriert. Das erhaltene Filtrat wurde ultrafiltriert (50 kDa PS-Membran) und das Permeat nachfolgend auf einen Kationenaustauscher gegeben und stufenweise pH-Wert abhängig eluiert. In den Eluaten enthaltene Peptide/Proteine wurden über RP-Chromatographie weiter aufgetrennt.

### HPLC Reinigung von antimikrobiellen Peptiden aus Thymusextrakt

Für die Reinigung von antimikrobiellen Peptiden aus Thymusextrakt können verschiedene HPLC Trennverfahren kombiniert werden, um eine möglichst reine Darstellung über eine optimale Trennung zu erzielen und inaktive, unerwünschte Bestandteile abzutrennen. Die jeweiligen Proben, die nach jedem Trennungsschritt entstehen, werden auf ihre antimikrobielle Aktivität gegen *Escherichia coli* mit Hilfe des Radial-Diffusions-Hemmtests untersucht (siehe Beispiel 2). Empfehlenswert für die Reinigung ist die Kombination von mindestens zwei Reverse Phase Chromatographieschritten und der Einsatz einer Kationenaustausch-HPLC-Trennung. In dem Biotest sollte die jeweilige Probe salzarm eingesetzt werden, um ein möglichst optimales Screeningergebnis zu erhalten.
Der erste Trennschritt wurde mit Hilfe einer Reverse Phase RP C15 Säule (10 x 15 cm, 15 µm, Pharmacia Biotech, Freiburg, Deutschland) durchgeführt.Puffer A: 10 mM HCl; Puffer B: 10 mM HCl / 80 % Acetonitril / 20 % bidestilliertes Wasser; Gradient: 1 % B / min, 60 Minuten; Fluss: 130 ml/min; Detektion: 280 nm

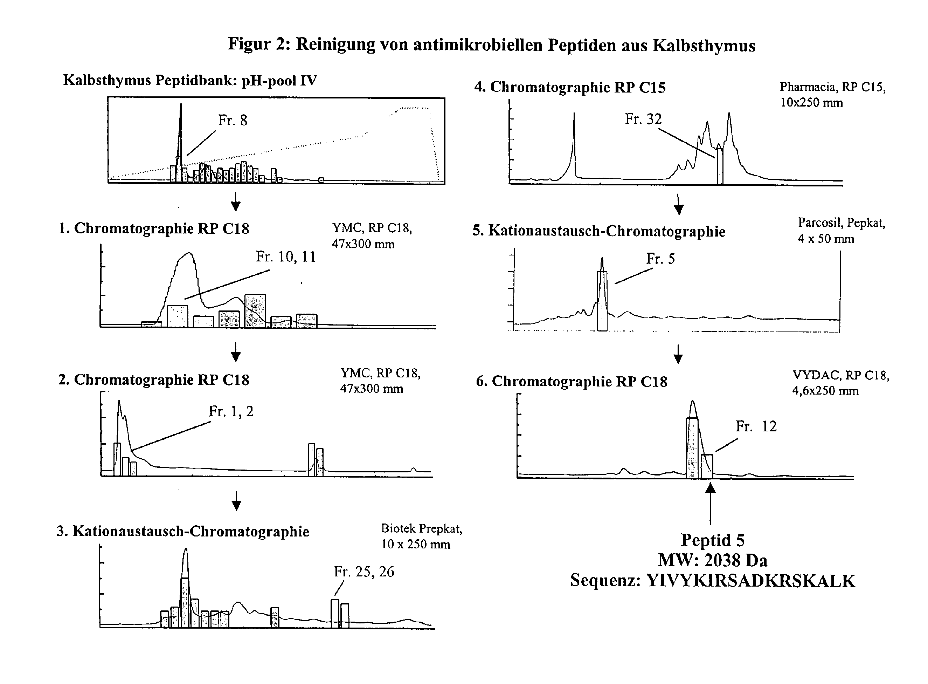

Das Chromatogramm ist in Figur 2 (pH-pool IV) dargestellt. Die Balken geben die antimikrobielle Aktivität der erhaltenen Fraktionen an.

Rechromatographie von Fraktion 8 mit einer YMC C18 Säule (47 x 300 mm, 15 µm, 300 Å, Waters, Milford, MA, USA). Puffer A: 10 mM HCl; Puffer B: 10 mM HCl / 80 % Acetonitril / 20 % bidestilliertes Wasser; Gradient: 10 - 60 % B in 50 min; Fluß: 40 ml/min; Detektion: 280 nm Das Chromatogramm ist in Figur 2 (1. Chromatographie) dargestellt. Die Balken geben die antimikrobielle Aktivität der erhaltenen Fraktionen an.
Rechromatographie von Fraktionen 10 und 11 mit einer YMC C18 Säule (47 x 300 mm, 15 µm, 300 Å, Waters, Milford, MA, USA). Puffer A: 20 % Methanol in 10 mM HCl; Puffer B: Methanol in 10 mM HCl; Gradient: 10 - 50 % B in 57 min; Fluß: 30 ml/min; Detektion: 280 nm Das Chromatogramm ist in Figur 2 (2. Chromatographie) dargestellt. Die Balken geben die antimikrobielle Aktivität der erhaltenen Fraktionen an.

Rechromatographie von Fraktionen 1 und 2 des vorangegangenen Trennschrittes mit Hilfe eines Kationenaustauschers.
Säule: Biotek Prepkat, 10 x 125 mm, 7 µm, 1000 Å, Biotek, Heidelberg, Deutschland
Puffer A: 50 mM Kaliumdihydrogenphosphatpuffer, pH 3,0; Puffer B: Puffer A mit 1,5 M KCl
Gradient: 10 - 60 % B in 50 min; Fluss: 2 ml/min; Detektion: 230 nm
Das Chromatogramm ist in Figur 2 (3. Chromatographie) dargestellt. Die erhaltenen Fraktionen wurden einzeln mit einem Reverse-Phase Chromatographieschritt entsalzt, bevor sie dem Test auf antimikrobielle Aktivität zugeführt wurden. Die Balken geben die antimikrobielle Aktivität der erhaltenen Fraktionen an.

Rechromatographie von Fraktionen 25 und 26 mit einer RP C15 Säule (10 x 250 mm, 15 µm, 120 Å, Pharmacia Biotech, Freiburg, Deutschland). Puffer A: 0,1 % TFA; Puffer B: 80 % Acetonitril in 0,085 % TFA; Gradient: 20 - 50 % B in 60 min; Fluß: 1,8 ml/min; Detektion: 214 nm Das Chromatogramm ist in Figur 2 (4. Chromatographie) dargestellt. Die Balken geben die antimikrobielle Aktivität der erhaltenen Fraktionen an.

Rechromatographie von Fraktion 32 des vorangegangenen Trennschrittes mit Hilfe eines Kationenaustauschers.
Säule: Parcosil Pepkat, 4 x 50 mm, 5 µm, 300 Å, Biotek, Heidelberg, Deutschland
Puffer A: 10 mM Natriumphosphatpuffer pH 7,2; Puffer B: Puffer A mit 1 M NaCl
Gradient: 0 - 60 % B in 60 Minuten; Fluss: 0,75 ml/min; Detektion: 214 nm.

Die erhaltenen Fraktionen wurden einzeln mit einem Reverse-Phase Chromatographieschritt entsalzt, bevor sie dem Test auf antimikrobielle Aktivität zugeführt wurden. Das Chromatogramm ist in Figur 2 (5. Chromatographie) dargestellt. Die Balken geben die antimikrobielle Aktivität der erhaltenen Fraktionen an.
Rechromatographie von Fraktion 5 mit einer Vydac RP C18 Säule (4,6 x 250 mm, 5 µm, 100 Å, Hesperia, CA, USA). Puffer A: 0,1 % TFA; Puffer B: 80 % Acetonitril in 0,085 % TFA; Gradient: 20 - 50 % B in 60 min; Fluss: 0,75 ml/min; Detektion: 214 nm Das Chromatogramm ist in Figur 2 (6. Chromatographie) dargestellt. Die Balken geben die antimikrobielle Aktivität der erhaltenen Fraktionen an.
Durch Massenspektrometrie und Aminosäuresequenzierung wurde das folgende Peptid identifiziert:
Fraktion 12 enthielt das reine, antimikrobiell wirksame Peptid 5 mit der Aminosäuresequenz: YIVYKIRSADKRSKALK Seq ID No. 5

### Beispiel 3

### Nachweis der antimikrobiellen Aktivität auf E. coli

Beim Radial-Diffusions-Hemmtest wird die antimikrobielle Wirkung von Probelösungen, hier HPLC Fraktionen, auf in Agarose suspendierte Bakterien, hier *E*. *coli*, untersucht. Dazu wurden die HPLC-Fraktionen lyophilisiert und in bidestilliertem Wasser aufgenommen. Zum Test ihrer antimikrobiellen Aktivität wurde wie folgt verfahren: zu dem bei 37 °C inkubierten Medium (300 mg/l Tryptic Soy Broth, 0,8 % (w/v) NuSieve GTG Agarose, 0,02 % (v/v) Tween 20 in 10 mM Natriumphosphatpuffer pH 7,2) wurde eine Bakteriensuspension (in LB-Medium), die eine optische Dichte von 1,0 bei einer Wellenlänge von 600 nm aufwies, gegeben. Nach Abkühlen der Agaroselösung in Petrischalen und Aushärtung bei 4 °C wurden steril Kavitäten mit einem Durchmesser von 3 mm in die feste Agarose gestanzt. In die Kavitäten wurden 10 µl Probelösung gegeben und die bestückten Platten bei 37 °C für 16 h inkubiert. Zur Auswertung der antimikrobiellen Aktivität der Proben wurden die entstandenen Hemmhöfe vermessen. Es besteht eine lineare Korrelation zwischen dem Logarithmus der Antibiotikakonzentration und den Hemmhofradien. Zum Vergleich antimikrobieller Aktivität verschiedener Fraktionen wurden entsprechend der Arbeit von Lehrer (Lehrer et al., J Immun Methods, Vol 137, S. 167, 1991) die Hemmhofradien in Einheiten umgerechnet. Dabei entspricht 1 Unit einem Hofradius von 0,1 mm.

### Beispiel 4

### Antimikrobielle Wirkung der erfindungsgemäßen Peptide gegen Bakterien und Hefen

Die antimikrobielle Aktivität der erfindungsgemäßen Peptide wurde unter Anwendung der Bestimmung der minimalen Hemmstoffkonzentration (MIC) gegen verschiedene Keime, wie Gram-positive und Gram-negative Bakterien und Hefen, untersucht. Die minimale Hemmstoffkonzentration bezeichnet die Peptidkonzentration, die minimal erforderlich ist, um das Wachstum von Mikroorganismen nach einer Inkubationszeit von 18 ± 2 h vollständig zu hemmen. Diese Quantifizierung der antimikrobiellen Aktivität ist mit chemisch synthetisierten Peptiden (Peptidsequenzen 1 - 5) gegen nicht pathogene sowie human pathogene Erreger durchgeführt worden. Die minimalen Hemmkonzentrationen sind in der nachfolgenden Tabelle in [µg/ml] angegeben:

| Peptide | 1 | 2 | 3 | 4 | 5 | 6 | 7 | 8 | 10 |
|---|---|---|---|---|---|---|---|---|---|
| Bacillus subtilis ATCC 6051 | 100 | 7.8 | 62.5 | 62.5 | 1.95 | 25 | 3,9 | nd | 20 |
| Micrococcus luteus ATCC 9341 | 200 | 31.25 | 31.25 | 200 | 6.25 | 50 | 25 | 31.2 5 | 18.7 5 |
| Staphylococcus aureus ATTC 25923 | 200 | nd | nd | >200 | 25 | 100 | 25 | 100 | 200 |
| Staphylococcus carnosus TM 300 | 100 | 12.5 | 40 | 50 | 6.25 | 25 | 15,6 | 12.5 | 37.5 |
| Escherichia coli ATTC 25922 | 200 | nd | nd | >200 | 6.25 | 50 | 12,5 | >20 0 | 100 |
| Escherichia coli BL21 (DE3) | 100 | 7.8 | 62.5 | 100 | 1.95 | 6,2 | 3,9 | >25 0 | 9.4 |
| Saccharomyces cerevisiae ATCC 9763 | 100 | 100 | 50 | 100 | 3.125 | 31,3 | 25 | >25 0 | 25 |
| Salmonella typhimurium ATCC 13311 | nd | nd | nd | nd | 10 | nd | nd | nd | nd |
| Pseudomonas aeruginosa clinical isolate | > 200 | 31.25 | > 200 | nd | 62.5 | nd | nd | nd | nd |
| Klebsiella pneumoniae ATCC 10031 | 300 | nd | nd | >300 | 150 | 75 | 18,75 | 100 | 25 |
| Mycobacterium ranae ATCC 110 | nd | nd | nd | nd | 30 | nd | nd | nd | nd |

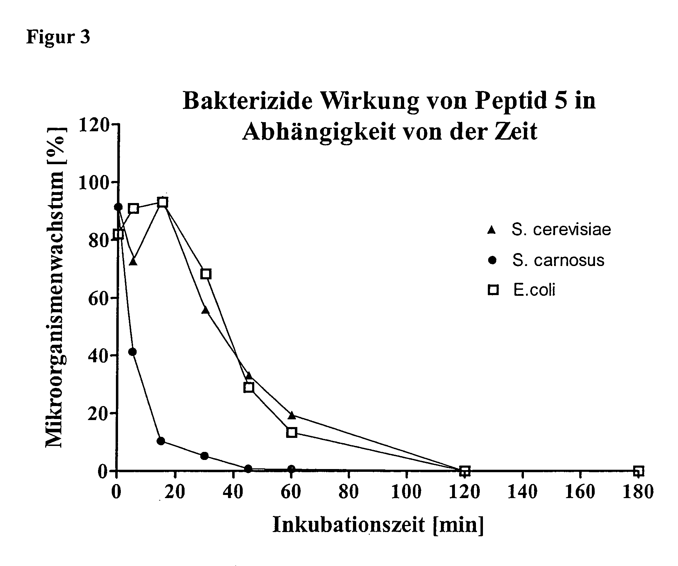

Die erfindungsgemäßen Peptide inhibieren effektiv sowohl das Wachstum von Gram-positiven und Gram-negativen Bakterien als auch von Hefen. Human pathogene Erreger, wie *Pseudomonas aeruginosa*, *Salmonella typhimurium* und *Klebsiella pneumoniae* werden ebenfalls effektiv abgetötet. Versuche zur Wirkkinetik ergaben, dass eine vollständige Abtötung der Keime bereits nach 2 h Inkubation erfolgte (siehe Figur 3).

## Patentansprüche

1. Peptid mit der Aminosäuresequenz
NH₂- VCVLAHHFGKEFTPPVQAAYQKWAGVANALAHKYH - COOH
sowie die amidierten, acetylierten, sulfatierten, phosphorylierten, glycosylierten, oxydierten Derivate und Fragmente, die aus der Aminosäuresequenz abgeleitet werden mit antimikrobieller Wirkung, insbesondere solche Fragmente, die durch konservativen Austausch oder Deletionen von Aminosäuren erhalten wurden.

2. Verfahren zur Herstellung der Peptides gemäß Anspruch 1 durch Aufreinigung aus menschlichen oder tierischen Gewebeextrakten oder aus menschlichen oder tierischen Körperflüssigkeiten, durch Proteolyse aus korrespondierenden Vorläuferproteinen, durch Expression in prokaryontischen oder eukaryontischen Organismen und durch chemische Synthese.

3. Arzneimittel, enthaltend mindestens ein Peptid gemäß Anspruch 1 als aktiven Wirkstoff zusammen mit pharmazeutisch üblichen Hilfs- und Zusatzstoffen.

4. Nahrungsmittel, enthaltend Peptide gemäß Anspruch 1 zusammen mit Nähr- und Zusatzstoffen.

5. Verwendung der Peptide gemäß Anspruch 1 in reiner Form als antibiotisches Pharmakon zur Behandlung von mikrobiell ausgelösten Erkrankungen.

6. Verwendung der Peptide gemäß Anspruch 1 in geeigneter Form, zubereitet für Infusionen, Salben, Tabletten, Sprays, "slow release"-Kapseln und ähnlichen Präparationen, als antibiotisches Pharmakon zur Behandlung von mikrobiell ausgelösten Erkrankungen.

7. Verwendung der Peptide gemäß Anspruch 1 zur Herstellung eines Arzneimittels zur Behandlung von Erkrankungen des menschlichen Organismus mit Beteiligung des Magen-Darm-Traktes, der Atemwege und des Urogenitaltraktes, und/oder zur Behandlung von Erkrankungen des menschlichen Organismus mit Beteiligung des Integumentes und seiner Anhangsdrüsen.

8. Verwendung der Peptide gemäß Anspruch 1 zur Herstellung eines Arzneimittels zur Behandlung von durch mikrobielle Fehlbesiedlung bedingten Erkrankungen, verursacht durch Bakterien, Pilze, Hefen, Protisten, Viren, Mycoplasmen, Filarien und/oder Plasmodien.

9. Verwendung nach Anspruch 8, **dadurch gekennzeichnet, dass** die mikrobiellen Fehlbesiedlungen Infektionen, Entzündungen, mikrobiell induzierte Tumoren, mikrobiell bedingte degenerative Erkrankungen, Durchfallerkrankungen, Koliken, Abweichungen der Mund-, Darm- und Vaginalflora und/oder Karies sind.

10. Verwendung der Peptide gemäß Anspruch 1 als Konservierungsstoff von Lebens- und Futtermitteln und/oder als Hilfsstoff bei industriellen Gärprozessen, insbesondere bei der Bierherstellung, bei der Joghurtproduktion usw., um die vorliegende Bakterienflora zu modifizieren.


SEQUENZPROTOKOLL

<110> IPF Pharmaceuticals GmbH

<120> Verfahren zur Gewinnung und Anwendung von antibiotisch
      wirksamen Peptiden zur Behandlung von
      Infektionskrankheiten

<130> IPF

<140>
<141>

<160> 12

<170> PatentIn Ver. 2.1

<210> 1
<211> 17
<212> PRT
<213> mammalian

<400> 1
Trp Gln Lys Met Val Thr Ala Val Ala Ser Ala Leu Ser Ser Arg Tyr
  1               5                  10                  15

His

<210> 2
<211> 31
<212> PRT
<213> mammalian

<400> 2
Gly Lys Val Lys Val Gly Val Asn Gly Phe Gly Arg Ile Gly Arg Leu
  1               5                  10                  15

Val Thr Arg Ala Ala Phe Asn Ser Gly Lys Val Asp Ile Val Ala
             20                  25                  30

<210> 3
<211> 36
<212> PRT
<213> mammalian

<400> 3
Val Cys Val Leu Ala His His Phe Gly Lys Glu Phe Thr Pro Pro Val
1               5                   10                  15

Gln Ala Ala Tyr Gln Lys Val Val Ala Gly Val Ala Asn Ala Leu Ala
            20                  25                  30

His Lys Tyr His
        35

<210> 4
<211> 19
<212> PRT
<213> mammalian

<400> 4
Lys Ala Gly Ala His Leu Lys Gly Gly Ala Lys Arg Val Ile Ile Ser
1               5                   10                  15

Ala Pro Ser

<210> 5
<211> 17
<212> PRT
<213> mammalian

<400> 5
Tyr Ile Val Tyr Lys Ile Arg Ser Ala Asp Lys Arg Ser Lys Ala Leu
1               5                   10                  15

Lys

<210> 6
<211> 23
<212> PRT
<213> mammalian

<400> 6
Lys Ala Ala Ser Lys Lys Ala Pro Ser Lys Ala Ser Pro Asn Pro Arg
1               5                   10                  15

Ser Gly Thr Arg Arg Ser Pro
            20

<210> 7
<211> 52
<212> PRT
<213> mammalian

<400> 7
Lys Ala Ala Ser Lys Lys Ala Pro Ser Lys Ala Ser Pro Asn Pro Arg
1               5                   10                  15

Ser Gly Thr Arg Arg Ser Pro Arg Ile Ala Thr Thr Ala Ala Ser Ala
            20                  25                  30

Ala Thr Ala Ala Ala Ser Ala Ala Ala Thr Thr Pro Arg Ala Lys Gly
        35                  40                  45

Lys Ala Lys His
    50

<210> 8
<211> 26
<212> PRT
<213> mammalian

<400> 8
Cys Ala Ile His Ala Lys Arg Val Thr Ile Met Pro Lys Asp Ile Gln
1               5                   10                  15

Leu Ala Arg Arg Ile Arg Gly Glu Arg Ala
            20                  25

<210> 9
<211> 31
<212> PRT
<213> mammalian

<400> 9
Leu Ser Lys Gly Leu Ile Lys Leu Val Ser Lys His Arg Ala Gln Val
1               5                   10                  15

Ile Tyr Thr Arg Asn Thr Lys Gly Gly Asp Ala Pro Ala Ala Gly
            20                  25                  30

<210> 10

<211> 39
<212> PRT
<213> mammalian

<400> 10
Gln Gln Lys Gln Gln Lys Gly Arg Gly Met Gly Gly Ala Gly Arg Gly
1               5                   10                  15

Val Phe Gly Gly Arg Gly Arg Gly Gly Ile Pro Gly Thr Gly Arg Gly
            20                  25                  30

Gln Pro Glu Lys Lys Pro Gly
        35

<210> 11
<211> 57
<212> PRT
<213> mammalian

<400> 11
Leu Lys Ser Leu Val Ser Lys Gly Thr Leu Val Gln Thr Lys Gly Thr
1               5                   10                  15

Gly Ala Ser Gly Ser Phe Lys Leu Asn Lys Lys Ala Ala Thr Gly Glu
            20                  25                  30

Ala Lys Pro Lys Gly Lys Lys Ala Gly Ala Ala Lys Pro Lys Lys Ala
        35                  40                  45

Ala Gly Ala Ala Lys Lys Pro Lys Lys
    50                  55

<210> 12
<211> 104
<212> PRT
<213> mammalian

<400> 12
Ser Ser Gly Glu Glu Val Val Ile Pro Gln Lys Lys Gly Lys Lys Ala
1               5                   10                  15

Ala Thr Thr Pro Ala Lys Lys Met Val Val Ser Pro Thr Lys Lys Val
            20                  25                  30

Ala Val Ala Thr Pro Ala Lys Lys Ala Val Val Thr Pro Gly Lys Lys
        35                  40                  45

Ala Ala Ala Met Pro Ala Lys Lys Thr Val Thr Pro Ala Lys Ala Val
     50                  55                  60

Val Thr Pro Gly Lys Lys Gly Ala Thr Pro Gly Lys Ala Val Val Ala
 65                  70                  75                  80

Thr Ser Gly Lys Lys Gly Ala Ala Thr Pro Gly Lys Gly Ala Lys Asn
                 85                  90                  95

Gly Lys Asn Ala Lys Lys Glu Asp
            100